# EUROPEAN PATENT SPECIFICATION

(11) **EP 2 883 541 B1**
(45) Date of publication and mention of the grant of the patent: **17.08.2016**
(21) Application number: 14187335.6
(22) Date of filing: 01.10.2014
(51) Int. Cl.: A61K 31/205, A61K 31/4166, A61K 31/728, A61P 17/02, A61K 9/00, A61L 15/28, A61L 15/44

(54) **Composition for topical use comprising hyaluronic acid**
Hyaluronsaüre enthaltende Zusammensetzung zur topischen Anwendung
Composition à usage topique comprenant l'acide hyaluronique

(30) Priority: 13.12.2013 IT RM20130686
(43) Date of publication of application: 17.06.2015
(73) Proprietor: Welcare Industries S.p.A., 05018 Orvieto (IT)
(72) Inventor: De Bernardini, Franco, 05020 Montecchio TR (IT); Lazzarotto, Fulvia, 00044 Frascati RM (IT); Zaru, Marco, 09128 Cagliari (IT)
(74) Representative: Germinario, Claudio

(56) References cited:
- DE-U1-202006 002 355
- DE-U1-202013 005 193
- US-A1- 2006 193 789
- US-A1- 2010 068 161

## Description

The present invention relates to new compositions for topical use comprising hyaluronic acid and liposomes comprising Allantoin and Acetyl-Carnitine. The present invention further relates to the methods for the preparation thereof and to the use thereof in the dermatological field, in particular to the use in the treatment of radiodermatitides.

### STATE OF PRIOR ART

The skin irritation caused by the ionizing radiations causes the phenomenon of radiodermatitides. Several of the known therapies for this disease do not result to be effective and often have disadvantages linked for example to the type of formulation.

Some of the compositions known for the use in the treatment of radiodermatitides and skin irritations in general include phospholipidic vesicles able to carry in the deepest layers of skin the active ingredients encapsulated therein wherein the active principle can easily explicit the action thereof. Such phospholipidic vesicled formations, better known with the term of Liposomes, have been described in literature for these potentialities thereof, but they have been hardly successful in the development of products considering their chemical-physical instability. In fact, these compositions often comprise surfactants and/or emulsifiers as stabilizing agents.

Recently the capability of the hyaluronic acid of intervening positively on the processes linked to the skin lesion has been highlighted, in particular it has been observed that not only it is about the fact that the activity is mediated by the hydrating and lenitive power carried out by the polymer, but even an action on the inflammatory process is added thereto (Vincenzo Liguori et. al. *"Double-Blind randomized clinical study comparing Hyaluronic acid cream in patient treated with radiotherapy*").

US2006/0193789 discloses a foamable composition comprising hyaluronic acid and allantoin for treating skin inflammation. Therefore it desirable to provide new compositions in the dermatological field effective in the treatment of radiodermatitides and skin irritations in general.

### SUMMARY OF THE INVENTION

The present invention provides a new composition for topical use which has resulted to be extremely effective in the prevention and treatment of radiodermatitides and skin irritations in general. Furthermore, the composition according to the present invention does not require the use of additional surfactants, emulsifiers or thickeners.

A first subject of the present invention is a composition for topical use comprising hyaluronic acid and liposomes comprising Allantoin and Acetyl-Carnitine.

A second subject of the present invention is a composition comprising hyaluronic acid and liposomes comprising Allantoin and Acetyl-Carnitine for topical use in the prevention and treatment of radiodermatitides, erythema, skin inflammation and skin lesions.

A third subject of the present invention is a method for preparing the compositions mentioned above comprising a passage of dispersing liposomes comprising Allantoin and Acetyl-Carnitine in a composition comprising hyaluronic acid.

A fourth subject of the present invention is a dressing for a dermatological treatment comprising a composition comprising hyaluronic acid and liposomes comprising Allantoin and Acetyl-Carnitine

Other advantages, together with the features and the use modes of the present invention, will result evident from the following detailed description of some preferred embodiments thereof, shown by way of example and with limitative purpose.

### BRIEF DESCRIPTION OF THE FIGURES

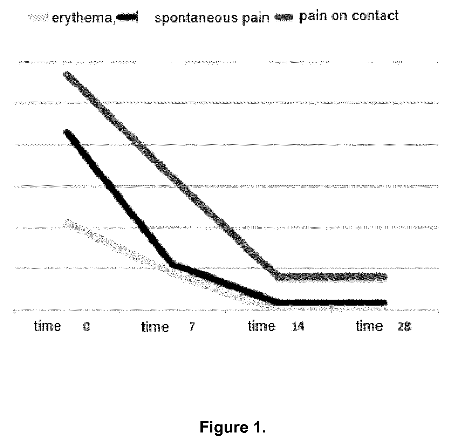

Figure 1. Figure 1 shows the results obtained by treating a group of patients having radiodermatitides with the composition of the present invention according to the embodiment of example 1.

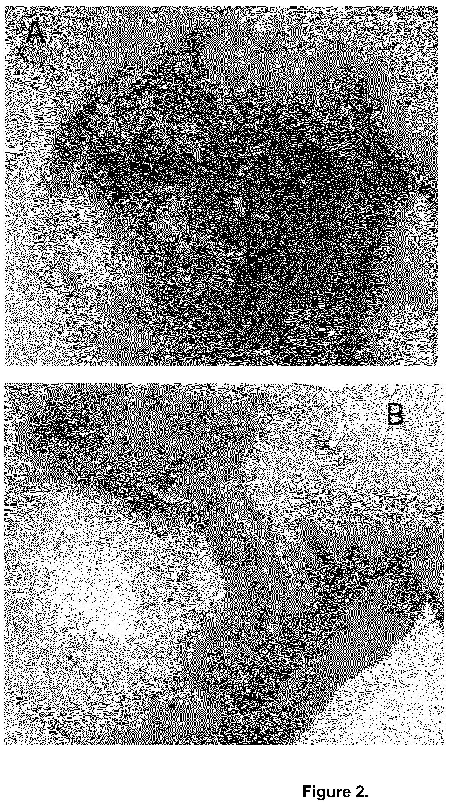

Figure 2. Figure 2 shows the improvement of the wound and of the perilesional skin of a patient after 3 weeks of treatment with the composition of the present invention according to the embodiment of example 1 (Figure 2A before treatment, Figure 2B after treatment).

### DETAILED DESCRIPTION OF THE INVENTION

Compositions for topical use comprising hyaluronic acid and liposomes comprising Allantoin and Acetyl-Carnitine are subjects of the present invention.

Under the expression "liposomes comprising Allantoin and Acetyl-Carnitine" in the present description it is to be meant that the compositions will comprise liposomes containing Allantoin and liposomes containing Acetyl-Carnitine and/or liposomes containing both Allantoin and Acetyl-Carnitine.

According to an embodiment the composition will include between 0.5 and 4% by weight of hyaluronic acid. Hyaluronic acid with different molecular weights, for example between 200 and 2000 KDa, could be used, optimum results in terms of formulation and efficiency could be obtained by using hyaluronic acid between 1000 and 2000 KDa, for example 1700 KDa. Mixtures of hyaluronic acid with different molecular weights, for example between 1 and 2% of hyaluronic acid with 200-400 KDa and between 1 and 2% of hyaluronic acid with 1000-2000 KDa, could even be used.

The presence of hyaluronic acid together with the other active components results to be amplified by the used formulative technology the role thereof is at the same time that of being an active and even structural ingredient for the formulation.

According to an embodiment in the hyaluronic acid-based composition, the phospholipidic vesicles containing the function active Allantoin and acetyl carnitine are dispersed. The phospholipidic vesicular formations, denominated liposomes are constituted by double phospholipidic layers (Phosphatidylcholine) dispersed in an aqueous medium which have then features of total atoxicity and biocompatibility. Their function is to carry the active ingredient through the skin surface layers and in particular through the corneous layer constituting the main barrier to the skin permeation. This function thereof of topic *"enhances"* allows the carried active ingredients to reach the skin layers wherein their activity can really explicit with important consequences on the final effects of the composition.

The hyaluronic acid-based composition has been enriched by means of selecting two active ingredients, Allantoin and Acetyl-Carnitine.

Allantoin (IUPAC name 2,5-Dioxo-4-imidazolidinyl-urea) is an important promoter of the epithelial regeneration process and of proliferation of the epidermic cells and a good modulator of the natural desquamation process of the corneous layer and further shows anti-irritant properties thanks to its capability of creating complexes and neutralizing several irritating agents. Allantoin could be for example in a concentration between 0.5 and 1.5 % by weight of the composition.

Acetyl-Carnitine (or even acetyl-L-carnitine, often shortened as ALC or ALCAR, IUPAC name (R)-3-acetyloxi-4-trimethylammonium-butanoate) is a metabolic intermediate deriving from L-carnitine, thereof it is the acetylated form with capability of intervening in the phase of cellular oxidative stress. Acetyl-Carnitine could be for example in a concentration between 0.5 and 1.5 % by weight of the composition.

The compositions could further comprise one or more compounds commonly used in the formulations for topical use such as for example excipients and preservatives.

The combination and consequent synergy between the components of the composition of the present invention and the particular formulative approach allows avoiding formulative adjuvants such as emulsifiers, thickeners and surfactants or other not suitable and contraindicated components, due to the high allergenic potential thereof. According to an embodiment the composition is then free of emulsifiers and/or thickeners and/or surfactants.

The compositions preferably will be in the form of a hydrophilic gel for example, a hyaluronic acid-based aqueous gel optionally comprising even glycerol and one or more preservatives.

Dressings for dermatological use are also subject of the present invention, comprising a composition according to any one of the embodiments described above. Said dressings could be in any one of the forms chosen among gauze, patch, bandages or other medical devices apt to cover a skin lesion or a skin irritation in general. The material of the dressings could be in any pharmaceutically acceptable tissue. For example, according to some embodiments, the material could be made of natural, synthetic polymeric fibres or any other type of material or tissue used and known in the medical state of art to cover a wound or keep at least a therapeutic agent or pharmaceutical composition in contact to the patient's skin.

The compositions and the dressings according to any one of the embodiments described above will be advantageously used in the prevention and treatment of radiodermatitides (skin lesions caused by the action of ionizing radiations such as for example X rays and gamma rays or by radioactive substances, even designated as radiodermatitides), erythema, skin inflammation and skin lesions in general.

Examples are reported herebelow which have the purpose of better illustrating the properties of the compositions of the present invention and some specific embodiments, such examples are not to be considered in any way as a limitation of the previous description and of the subsequent claims.

### EXAMPLES

**Example 1 quantity per 100 g of product**

| INGREDIENT | QUANTITY GRAMS |
|---|---|
| Acetyl Carnitine (L) Hydrochloride | 0.5 |
| Allantoin | 0.5 |
| Glycerol | 4 |
| P90H (Phospholipon 90 H) | 0.3 |
| Sodium hyaluronate (1700 Kda) | 1.4 |
| Sodium hyaluronate (200-400 Kda) | 0.1 |
| Preservative KT | 0.038 |
| Water | 93.16 |

**Example 2 quantity per 100 g of product**

| INGREDIENT | QUANTITY GRAMS |
|---|---|
| Acetyl Carnitine (L) Hydrochloride | 0.8 |
| Allantoin | 0.5 |
| Glycerol | 5 |
| P90H (Phospholipon 90 H) | 0.4 |
| Sodium hyaluronate (1700 Kda) | 1.6 |
| Sodium hyaluronate (200-400 Kda) | 0.2 |
| Preservative KT | 0.038 |
| Water | 92.11 |

**Example 3 quantity per 100 g of product**

| DESCRIPTION | QUANTITY GRAMS |
|---|---|
| Acetyl Carnitine (L) Hydrochloride | 0.3 |
| Allantoin | 0.4 |
| Glycerol | 2 |
| P90H (Phospholipon 90 H) | 0.2 |
| Sodium hyaluronate (1700 Kda) | 1.4 |
| Sodium hyaluronate (200-400 Kda) | 0.1 |
| Preservative KT | 0.038 |
| Water | 95.562 |

### Example 4 preparation method

Heat the water at about 70-75°C, add Allantoin and keep under stirring until when the solution becomes limpid. Then, by switching off the source of heat, add Acetyl carnitine, glycerol and transfer the whole under turboemulsor. Add P90H and subsequently still under turbo-emulsion the correct quantity of half-finished product 2 and then, by reducing the turbo-emulsion and widening the planetary stirring, add the correct quantity of half-finished product 1.

Let stirring slowly for at least 2 hours, then make to rest 2-3 hours to remove air bubbles and vacuum work.

### Half-finished product 1

Gel of hyaluronic acid 1000-2000 Kda preferably 1700 Kda in concentration from 0.5 to 3% preferably from 1% to 2.5% more preferably 2%.

Dissolve gradually the Sodium hyaluronate in the suitable quantity of (sterile or depurated) water, add the preservative solution (preservative KT) and leave under vigorous, then slow, mechanical stirring for about 36 hours.

### Half-finished product 2

Gel of hyaluronic acid 100- 800 Kda preferably 200-400 Kda in concentration from 0.5% to 4% preferably 1% -2% more preferably 1.5%.

Dissolve gradually the Sodium hyaluronate in the (sterile or depurated) water, add the preservative solution (preservative KT) and leave under slow mechanical stirring for about 18 hours.

### 5. Clinic experiment: treatment of the radiodermatitides with the preparation according to example 1

The radiodermatitides are a skill insult due to the effect of the ionizing radiations for therapeutic purposes. Such radiations are responsible, first of all, for an erythematosus area reaching often the exudative phase, passing through the oedematous phase. It is then an erythematosus manifestation which causes a lesion involving the skin entirely and causing pain, sometimes an incoercible pain, refractory to any antalgic, both topic and pharmacological, treatment by systematic route.

The areas lesioned by ionizing radiations have been treated, mainly in the mammary seat, but even in sacro-gluteal seat. The product application has been daily and it consisted of a whole covering of the area to be treated (radiodermatitic erythema) by means of a thin film of product; in case of presence of skin exulceration due to a dermal erosion or possible ulcerated neoplasia, the dressing protocol has not been changed, therefore the product has been considered a dressing supporting the radiodermatitic erythematous phase.

The main target we have set is to reduce pain (evaluated according to VAS-Visual Analogue Scale), and then an improvement in the life quality of patients; a secondary target has been considered the erythema reduction, meant both as reduction in the intensity of the erythema itself, and as decrease in the area involved by the radiodermatitic process. Even the thermal variation of the skin area victim of inflammation (iper or normo thermotact) has been considered as indicator of the lenitive/healing effect of the product. At last, the resolution of possible micro/macrolesions existing in the radiodermatitic area has been evaluated.

The results have been absolutely positive, above all from the subjective point of view of the patient who has found very quick advantage from the antalgic point of view, by involving a significant reduction in pain, decreased to negligible levels or even disappeared, in a time elapse variable from 3 to 10 days. Even the erythema has considerably improved, both in terms of extension and intensity, whereas the perilesional skin (when it has ulcerative lesions) has shown quick and effective signs of regeneration/repair.

On a quality level, the skin, previously involved by the radiodermatitic process, after the treatment has shown in optimum status of vascularisation, hydratation and elasticity; in most cases the spontaneous pain has almost disappeared, only persisting a certain ache at the touch; even the local hyperthermia has disappeared in a very short time lapse, by normalizing the thermotouch within 7-13 days. We inform that the stinging sensation characterizing the radiodermatitic pain has tended towards remission already after the first application of the product and that the patients have reported globally an important pain reduction already after 48 hours only as from the treatment beginning.

The analysis of the sofar obtained data shows that the product succeeds in reaching the derma wherein it carries out the antalgic action thereof, by setting at rest the herein localized nerve endings, thus reducing the stimulation thereof. The action on the erythema follows exactly the antalgic one, even if however the intervention on pain seems absolutely the most rapid one.

**Table 1 Results of experiment on radiodermatitides**

| | Week 1 | Week 2 | Week 3 |
|---|---|---|---|
| Pain | **-74,5 %** | **-95,4%** | **-95,4%** |
| Erythema | **-50%** | **-100%** | |
| Heat of skin | **-75%** | **-75%** | **-100%** |

### 6. Card for collecting data of some patients subjected to the experiment

| | | | | | | |
|---|---|---|---|---|---|---|
| Patient: K.L.(01) | MALE | FEMALE | | Date of birth: 02.04.1943 | | |
| Radiodermitis seat: perilesional mammary | | | | Date of recruitment: 06.122012 | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 04 | | Upon palpation: 07 | |

| | | | | | | |
|---|---|---|---|---|---|---|
| Date of check: 13.12.2012 | | | Product application frequency: Daily | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 02 | | Upon palpation: 04 | |

| | | | | | | |
|---|---|---|---|---|---|---|
| Date of check: 19.12.2012 | | Product application frequency: Daily | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 00 | | Upon palpation: 02 | |

| | | | | | | |
|---|---|---|---|---|---|---|
| Date of check: 09.01.2013 | | Product application frequency: Daily | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 00 | | Upon palpation: 00 | |

| | | | | | | |
|---|---|---|---|---|---|---|
| Date of check: | | Product application frequency: | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: | | Upon palpation: | |

| | | | | | | |
|---|---|---|---|---|---|---|
| Patient: P.R.D.(02) | MALE | FEMALE | | Date of birth: | | |
| Radiodermitis seat: perilesional mammary | | | | Date of recruitment: 14.01.2013 | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 08 | | Upon palpation: 10 | |

| | | | | | | |
|---|---|---|---|---|---|---|
| Date of check: 24.01.2013 | | Product application frequency: Daily | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 02 | | Upon palpation: 07 | |

| | | | | | | |
|---|---|---|---|---|---|---|
| Date of check: 01.03.2013 | | Product application frequency: Daily | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 00 | | Upon palpation: 01 | |

| | | | | | | |
|---|---|---|---|---|---|---|
| Date of check: 15.03.2013 | | Product application frequency: Daily | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 00 | | Upon palpation: 00 | |

| | | | | | | |
|---|---|---|---|---|---|---|
| Date of check | | Product application frequency | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous | | Upon palpation | |

| | | | | | | |
|---|---|---|---|---|---|---|
| Patient: C.M.(03) | MALE | FEMALE | | Date of birth: 24.03.1950 | | |
| Radiodermitis seat: perilesional mammary | | | | Date of recruitment: 15.012013 | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 08 | | Upon palpation: 08 | |

| Date of check: 15.02.2013 | | Product application frequency: Daily | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 01 | | Upon palpation: 04 | |

| Date of check: | | Product application frequency: | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: | | Upon palpation: | |

| Date of check: | | Product application frequency: | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: | | Upon palpation: | |

| Date of check: | | Product application frequency: | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: | | Upon palpation: | |

| | | | | | | |
|---|---|---|---|---|---|---|
| Patient: G.E. (04) | MALE | FEMALE | | Date of birth: 30.08.1942 | | |
| Radiodermitis seat: perilesional mammary | | | | Date of recruitment: 21.01.2013 | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 09 | | Upon palpation: 10 | |

| Date of check: 11.02.2013 | | Product application frequency: Daily | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 05 | | Upon palpation: 08 | |

| Patient: V.M. (05) | MALE | FEMALE | | Date of birth: 29.06.1954 | | |
|---|---|---|---|---|---|---|
| Radiodermitis seat: perilesional mammary | | | | Date of recruitment: 28.02.2013 | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 00 | | Upon palpation: 02 | |

| Date of check: 15.03.2013 | | Product application frequency: Daily | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 00 | | Upon palpation: 00 | |

| Date of check: | | Product application frequency: | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: | | Upon palpation: | |

| Date of check: | | Product application frequency: | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: | | Upon palpation: | |

| Date of check: | | Product application frequency: | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: | | Upon palpation: | |

| | | | | | | |
|---|---|---|---|---|---|---|
| Patient: C.A.(06) | MALE | FEMALE | | Date ofbirth: 14.03.1964 | | |
| Radiodermitis seat: Perilesional glutei | | | | Date of recruitment: 08.03.2013 | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 06 | | Upon palpation: 10 | |

| Date of check: 19.03.2013 | | Product application frequency: Daily/Upon each change | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous: 00 | | Upon palpation: 06 | |

| | | | | | | |
|---|---|---|---|---|---|---|
| Date of check | | Product application frequency | | | | |
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous | | Upon palpation | |

| Date of check | | Product application frequency | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous | | Upon palpation | |

| Date of check | | Product application frequency | | | | |
|---|---|---|---|---|---|---|
| ENTITY OF ERYTHEMA | | | | | | |
| Absent | Faint | Moderate | | Frank | | Severe |
| INSUDATION YES NO | | | THERMOTOUCH IPER ISO IPO | | | |
| PAIN according to Visual Analogue Scale (VAS) | | | Spontaneous | | Upon palpation | |

## Claims

1. A composition for topical use comprising hyaluronic acid and liposomes comprising Allantoin and Acetyl-Carnitine.

2. The composition according to claim 1 wherein said hyaluronic acid is in a concentration between 0.5 and 4% by weight of said composition.

3. The composition according to claim 1 or 2 wherein said hyaluronic acid has a molecular weight between 1000 and 2000 KDa, preferably 1700 KDa.

4. The composition according to any one of claims 1 to 3 in the form of a hydrophilic gel.

5. The composition according to any one of claims 1 to 4 free of surfactants and/or emulsifiers and/or thickeners.

6. The composition according to any one of claims 1 to 5 wherein said Allantoin and/or Acetyl-Carnitine is in a concentration between 0.5 and 1.5% by weight of said composition .

7. The composition according to any one of claims 1 to 6 for topical use in the treatment and prevention of radiodermatitides, erythema, skin inflammation and skin lesions.

8. A method for the preparation of a composition according to any one of claims 1 to 7 comprising a passage wherein liposomes comprising Allantoin and Acetyl-Carnitine are dispersed in a composition comprising hyaluronic acid.

9. A dressing for dermatological use comprising a composition according to any one of claims 1 to 7.

10. The dressing according to claim 9 in the form of gauze, patch, bandage or other medical devices apt to cover a wound or skin irritation.

## Patentansprüche

1. Zusammensetzung zur topischen Anwendung, umfassend Hyaluronsäure und Liposome, umfassend Allantoin und Acetylcarnitin.

2. Zusammensetzung nach Anspruch 1, wobei die Hyaluronsäure in einer Konzentration von zwischen 0,5 und 4 Gew.-% der Zusammensetzung vorliegt.

3. Zusammensetzung nach Anspruch 1 oder 2, wobei die Hyaluronsäure ein Molekulargewicht zwischen 1000 und 2000 kDa, vorzugsweise 1700 kDa hat.

4. Zusammensetzung nach einem der Ansprüche 1 bis 3 in Form eines hydrophilen Gels.

5. Zusammensetzung nach einem der Ansprüche 1 bis 4, frei von Tensiden und/oder Emulgatoren und/oder Verdickungsmitteln.

6. Zusammensetzung nach einem der Ansprüche 1 bis 5, wobei das Allantoin und/oder Acetylcarnitin in einer Konzentration zwischen 0,5 und 1,5% Gew.-% der Zusammensetzung vorliegt.

7. Zusammensetzung nach einem der Ansprüche 1 bis 6 zur topischen Anwendung bei der Behandlung und Vorbeugung von Radiodermatitis, Erythem, Hautentzündung und Hautläsionen.

8. Verfahren zur Herstellung einer Zusammensetzung nach einem der Ansprüche 1 bis 7, umfassend eine Passage, wobei Allantoin und Acetylcarnitin umfassende Liposome in einer Zusammensetzung dispergiert sind, die Hyaluronsäure umfasst.

9. Wundauflage zur dermatologischen Anwendung, die eine Zusammensetzung nach einem der Ansprüche 1 bis 7 umfasst.

10. Wundauflage nach Anspruch 9 in Form von Mull, Pflaster, Verband oder anderen Medizinprodukten, die zur Abdeckung einer Wunde oder von Hautreizung geeignet sind.

## Revendications

1. Composition à usage topique comprenant de l'acide hyaluronique et des liposomes comprenant de l'allantoïne et une acétyl-carnitine.

2. Composition selon la revendication 1 dans laquelle ledit acide hyaluronique est à une concentration entre 0,5 et 4 % en poids de ladite composition.

3. Composition selon la revendication 1 ou 2 dans laquelle ledit acide hyaluronique a un poids moléculaire entre 1000 et 2000 KDa, de préférence de 1700 KDa.

4. Composition selon l'une quelconque des revendications 1 à 3 sous la forme d'un gel hydrophile.

5. Composition selon l'une quelconque des revendications 1 à 4 exempte de tensioactifs et/ou d'émulsifiants et/ou d'épaississants.

6. Composition selon l'une quelconque des revendications 1 à 5 dans laquelle ladite allantoïne et/ou acétyl-carnitine est à une concentration entre 0,5 et 1,5 % en poids de ladite composition.

7. Composition selon l'une quelconque des revendications 1 à 6 à usage topique destinée à traiter et à prévenir la radiodermite, l'érythème, l'inflammation cutanée et les lésions cutanées.

8. Procédé de préparation d'une composition selon l'une quelconque des revendications 1 à 7 comprenant un passage dans lequel les liposomes comprenant l'allantoïne et l'acétyl-carnitine sont dispersés dans une composition comprenant l'acide hyaluronique.

9. Pansement à usage dermatologique comprenant une composition selon l'une quelconque des revendications 1 à 7.

10. Pansement selon la revendication 9 sous la forme d'une gaze, d'un patch, d'un bandage ou autres dispositifs médicaux aptes à recouvrir une lésion ou une irritation cutanée.
